(19) **Europäisches Patentamt / European Patent Office / Office européen des brevets**

(11) **EP 3 543 696 A2**

(12) # EUROPEAN PATENT APPLICATION
published in accordance with Art. 153(4) EPC

(43) Date of publication:
**25.09.2019 Bulletin 2019/39**

(51) Int Cl.:
***G01N 33/564*** *(2006.01)*

(21) Application number: **17869769.4**

(22) Date of filing: **14.11.2017**

(86) International application number:
**PCT/ES2017/070752**

(87) International publication number:
**WO 2018/087416 (17.05.2018 Gazette 2018/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **14.11.2016 AR P016103481**

(71) Applicants:
- **Consejo Nacional de Investigaciones Científicas Y Técnicas (CONICET)**
  **Ciudad Autónoma de Buenos Aires 1425 (AR)**
- **Fundación Articular**
  **Provincia de Buenos Aires B1878DVB (AR)**
- **Velasco Zamora, Benito Jorge**
  **03540 Alicante (ES)**

(72) Inventors:
- **VELASCO ZAMORA, Benito Jorge**
  **03540 Alicante (ES)**
- **DEWEY, Ricardo Alfredo**
  **Chascomús**
  **Buenos Aires 7130 (AR)**
- **CARREA, Alejandra**
  **La Plata 1900 (AR)**
- **PERONE, Marcelo Javier**
  **Buenos Aires C1425FQD (AR)**
- **PREISEGGER, Matías Adán**
  **Quilmes B1878DVB (AR)**

(74) Representative: **Trupiano, Federica**
**Torner, Juncosa I Associats, S.L.**
**Gran Via de les Corts Catalanes**
**669 BIS 1° 2a**
**08013 Barcelona (ES)**

(54) **METHOD FOR DETERMINING THE ACTIVITY OF AUTOIMMUNE DISEASES AND KIT**

(57) A method to determine the activity level of autoimmune diseases, the said method comprising a) to supply a biological sample; b) to determine the TβRII-A, TβRII-B and TβRII-Se isoforms level of the biological sample; c) calculate the activity level by performing the quotient between the TβRII-Se level and the level of the addition of TβRII-A y TβRII-B. The isoforms level can be measured by detecting the polypeptides of said isoforms or the mRNA of the isoforms in the isolated circulating mononuclear blood cells by means of, for instance, RT-qPCR.

Figure 1

EP 3 543 696 A2

SEQUENCE LISTING

<110>  Conicet, Fundación Articular y Universidad de San Martín

<120>  Método de diagnóstico y actividad de enfermedades autoinmunes y kit

<130>  Dr Dewey et. al.,

<160>  12

<170>  PatentIn version 3.5

<210>  1
<211>  218
<212>  DNA
<213>  Homo sapiens

<400>  1
gcacgttcag aagtcggtta ataacgacat gatagtcact gacaacaacg gtgcagtcaa      60

gtttccacaa ctgtgtaaat tttgtgatgt gagattttcc acctgtgaca accagaaatc     120

ctgcatgagc aactgcagca tcacctccat ctgtgagaag ccacaggaag tctgtgtggc     180

tgtatggaga aagaatgacg agaacataac actagaga                             218

<210>  2
<211>  21
<212>  DNA
<213>  Artificial Sequence

<220>
<223>  P3

<400>  2
gcacgttcag aagtcggtta a                                                21

<210>  3
<211>  22
<212>  DNA
<213>  Artificial Sequence

<220>
<223>  P2

<400>  3
tctctagtgt tatgttctcg tc                                               22

<210>  4
<211>  233
<212>  DNA
<213>  Homo sapiens

<400>  4
ctgtaatagg actgcccatc cactgagaca tattaataac gacatgatag tcactgacaa      60

caacggtgca gtcaagtttc cacaactgtg taaattttgt gatgtgagat tttccacctg     120

tgacaaccag aaatcctgca tgagcaactg cagcatcacc tccatctgtg agaagccaca     180

ggaagtctgt gtggctgtat ggagaaagaa tgacgagaac ataacactag aga          233


<210>  5
<211>  20
<212>  DNA
<213>  Artificial Sequence

<220>
<223>  P1

<400>  5
ctgtaatagg actgcccatc          20


<210>  6
<211>  137
<212>  DNA
<213>  Homo sapiens

<400>  6
gcacgttcag aagtcggtta ataacgacat gatagtcact gacaacaacg gtgcagtcaa          60

gtttccacaa ctgtgtaaat tttgtgatgt gagattttcc acctgtgaca accagaaatc          120

ctgcttctcc aaagtgc          137


<210>  7
<211>  21
<212>  DNA
<213>  Artificial Sequence

<220>
<223>  P4

<400>  7
gcactttgga gaagcaggat t          21


<210>  8
<211>  167
<212>  PRT
<213>  Homo sapiens

<400>  8

Met Gly Arg Gly Leu Leu Arg Gly Leu Trp Pro Leu His Ile Val Leu
1               5                   10                  15

Trp Thr Arg Ile Ala Ser Thr Ile Pro Pro His Val Gln Lys Ser Val
            20                  25                  30

Asn Asn Asp Met Ile Val Thr Asp Asn Asn Gly Ala Val Lys Phe Pro
        35                  40                  45

Gln Leu Cys Lys Phe Cys Asp Val Arg Phe Ser Thr Cys Asp Asn Gln
    50                  55                  60

```
Lys Ser Cys Met Ser Asn Cys Ser Ile Thr Ser Ile Cys Glu Lys Pro
65              70              75                       80


Gln Glu Val Cys Val Ala Val Trp Arg Lys Asn Asp Glu Asn Ile Thr
            85              90              95


Leu Glu Thr Val Cys His Asp Pro Lys Leu Pro Tyr His Asp Phe Ile
            100             105             110


Leu Glu Asp Ala Ala Ser Pro Lys Cys Ile Met Lys Glu Lys Lys Lys
            115             120             125


Pro Gly Glu Thr Phe Phe Met Cys Ser Cys Ser Ser Asp Glu Cys Asn
    130             135             140


Asp Asn Ile Ile Phe Ser Glu Glu Tyr Asn Thr Ser Asn Pro Asp Leu
145             150             155             160


Leu Leu Val Ile Phe Gln Val
                165


<210>    9
<211>    192
<212>    PRT
<213>    Homo sapiens

<400>    9

Met Gly Arg Gly Leu Leu Arg Gly Leu Trp Pro Leu His Ile Val Leu
1               5               10                      15


Trp Thr Arg Ile Ala Ser Thr Ile Pro Pro His Val Gln Lys Ser Asp
            20              25              30


Val Glu Met Glu Ala Gln Lys Asp Glu Ile Ile Cys Pro Ser Cys Asn
            35              40              45


Arg Thr Ala His Pro Leu Arg His Ile Asn Asn Asp Met Ile Val Thr
    50              55              60


Asp Asn Asn Gly Ala Val Lys Phe Pro Gln Leu Cys Lys Phe Cys Asp
65              70              75                      80


Val Arg Phe Ser Thr Cys Asp Asn Gln Lys Ser Cys Met Ser Asn Cys
            85              90              95


Ser Ile Thr Ser Ile Cys Glu Lys Pro Gln Glu Val Cys Val Ala Val
            100             105             110
```

Trp Arg Lys Asn Asp Glu Asn Ile Thr Leu Glu Thr Val Cys His Asp
        115                 120                 125

Pro Lys Leu Pro Tyr His Asp Phe Ile Leu Glu Asp Ala Ala Ser Pro
        130                 135                 140

Lys Cys Ile Met Lys Glu Lys Lys Lys Pro Gly Glu Thr Phe Phe Met
145                 150                 155                 160

Cys Ser Cys Ser Ser Asp Glu Cys Asn Asp Asn Ile Ile Phe Ser Glu
                165                 170                 175

Glu Tyr Asn Thr Ser Asn Pro Asp Leu Leu Leu Val Ile Phe Gln Val
            180                 185                 190

<210> 10
<211> 80
<212> PRT
<213> Homo sapiens

<400> 10

Met Gly Arg Gly Leu Leu Arg Gly Leu Trp Pro Leu His Ile Val Leu
1               5                   10                  15

Trp Thr Arg Ile Ala Ser Thr Ile Pro Pro His Val Gln Lys Ser Val
            20                  25                  30

Asn Asn Asp Met Ile Val Thr Asp Asn Asn Gly Ala Val Lys Phe Pro
        35                  40                  45

Gln Leu Cys Lys Phe Cys Asp Val Arg Phe Ser Thr Cys Asp Asn Gln
    50                  55                  60

Lys Ser Cys Phe Ser Lys Val His Tyr Glu Gly Lys Lys Lys Ala Trp
65                  70                  75                  80

<210> 11
<211> 19
<212> DNA
<213> Artificial Sequence

<220>
<223> GAPDH-F

<400> 11
gtcagtggtg gacctgacc                                                    19

<210> 12
<211> 20
<212> DNA

```
<213>  Artificial Sequence

<220>
<223>  GAPDH-R

<400>  12
tgagcttgac aaagtggtcg                                                    20
```

**Description**

Field of the Art

The present invention relates to a method to diagnose or determine, or both at the same time, the activity level of autoimmune diseases, the said method comprising a) to supply a biological simple; b) to determine the TβRII-A, TβRII-B and TβRII-Se isoforms level in the biological sample; c) calculate the activity level by performing the quotient between the TβRII-Se level and the level of the addition of TβRII-A y TβRII-B. The isoform level can be measured by detecting the polypeptides of said isoforms or the mRNA of the isoforms in the isolated circulating mononuclear blood cells by means of, for instance, RT-qPCR.

State of the Art

Rheumatoid arthritis is an autoimmune disease. It is believed that this kind of diseases occur because of abnormalities of the immune response, be it innate or adaptive, and very probably they have genetic or environmental components. A diagnosis of this kind of diseases is usually difficult, because their symptoms tend to be relatively non -specific. Consequently, no available blood analysis may exclude, with a degree of certainty, the possibility of an autoimmune disease in individuals showing said symptoms. As the best choice, the performance of a battery of trials and a time assessment by a specialized physician is required, in order to establish whether a patient actually has an autoimmune disease.

To date, there is no definite diagnostic trial confirming a RA diagnosis. Some tests can furnish subjective data that may increase diagnostic certainty and allow the follow up of the disease diagnosis.

Clinical methods currently used for the assessment and classification to assess rheumatoid arthritis (RA), both in clinical trials and in clinical practice, are not sensitive or reproducible enough. The clinical assessment method most frequently utilized is the DAS28, which has potential errors, among which are included the subjectivity of a clinical assessment, the presence of important changes in the joint count with poor repercussion on VSG levels, this marker having a significant weight within the score and the significant differences found in the qualification different observers make of a same patient.

At the present time there exist methods having higher sensitivity and specificity for the detection of joint inflammation, such as NMR and joint ultrasonography, the former being the most sensitive, but also being the most expensive and the one that requires most time for its implementation though, this rendering it a scarcely profitable technique for the periodical assessment needed in clinical practice. On the other hand, high-definition ultrasonography has an accuracy similar to resonance's for the identification of certain pathological changes. However there is no consensus about an echographic scale. Doubts exist about the reliability of fixed images acquisition and interpretation and there is no consensus regarding the assessment system for synovites and there do not exist compound scores using echographic evaluation as a part of patient assessment, to replace joint counts.

Argentine patent application P20130104170 (AR093466A19) discloses the detection of **TβRII-A y TβRII-B** isoforms in monocytes.

Assays that improve sensitivity and facilitate clinical assessments, in order to make therapeutic decisions, are needed.

Brief description of the present invention

A method to diagnose or determine, or both at the same time, the activity level of autoimmune diseases is provided, the said method comprising:

a) providing a biological sample;

b) determining the level of the TβRII-A, TβRII-B y TβRII-Se isoforms in that biological sample

c) calculating the activity level by performing the quotient between the TβRII-Se level and the level of the addition of TβRII-A and TβRII-B.

The biological sample may be isolated mononuclear circulating blood cells or the polypeptides of TβRII-A, TβRII-B and TβRII-Se isoforms. The level of isoforms can be measured by the detection of the polypeptides of said isoforms or the mRNA of the isoforms in the isolated circulating mononuclear blood cells. In a preferred embodiment, the level of mRNA in the isoforms is detected by means of RT-qPCR.

In the method the level of the polypeptides of the TβRII-A, TβRII-B and TβRII-Se isoforms can be measured, for example, by measuring the level of polypeptides in the sequences SEQ ID N° 8, SEQ ID N° 9 and SEQ ID N° 10 or in sequences having an identity of 90% at the least.

The present method can be used to diagnose or determine, or do both things at the same time with, the activity level of autoimmune diseases such as rheumatoid arthritis,systemic lupus erythematosus, systemic sclerosis, polydermatomyositis, vasculitis and seronegative spondyloarthropathies.

A kit is provided to diagnose or determine, or do both things at the same time, the activity level of autoimmune diseases. The said kit comprises:

i) specific primers to amplify the TβRII-A, TβRII-B and TβRII-Se isoforms,

ii) reverse transcriptase and Taq polymerase enzymes

iii) means to reveal said isoforms.

The kit may comprise means for the obtainment and isolation of blood mononuclear cells and means of revelation such as, for example, fluorescence, colorimetry and luminiscence.

Description of figures

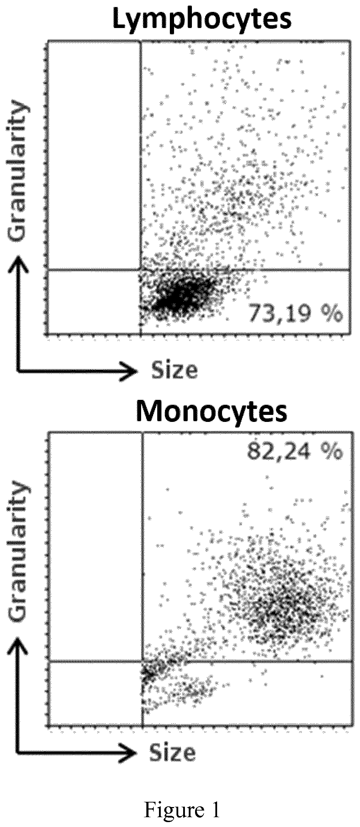

Figure 1 shows flow cytometry diagrams that represent populations of monocytes and lymphocytes isolated from the volunteer identified as AR#9. The respective purity percentages are shown.

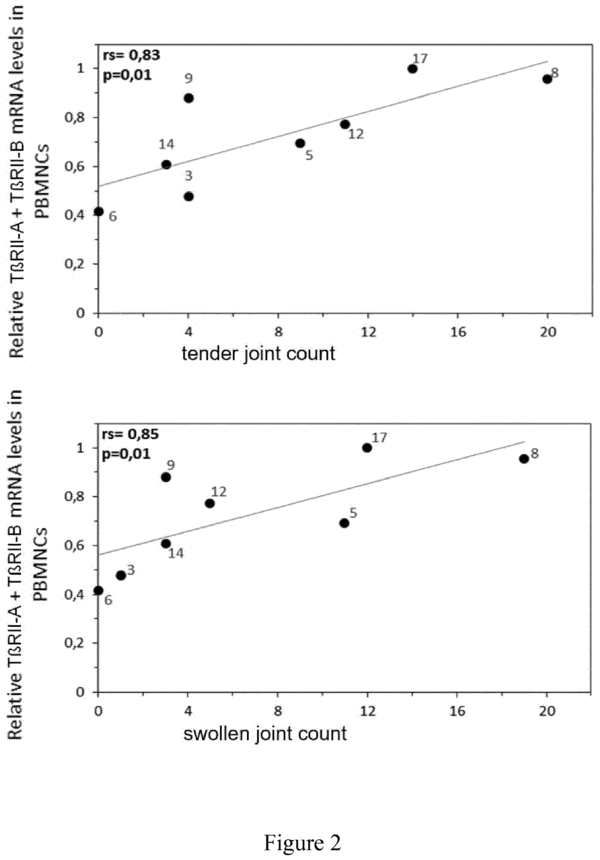

Figure 2 shows the graphs for the correlations that are positive between the mRNA level of TβRII-A and TβRII-B in PBMNCs (addition of relative abundance of monocytes and lymphocytes) and clinical determinations of the tender joint count (TJC) and the swollen joint count /SJC). The numbers correspond to the number assigned to each volunteer involved in the study. The analysis of the correlations was carried out by employing Spearman's test, with the use of the OriginPro 8.5.1 software (Origin Lab Corporation, Northampton, MA, USA). Values of $p < 0.05$ were considered to have statistical significance. rs: Spearman's correlation coefficient.

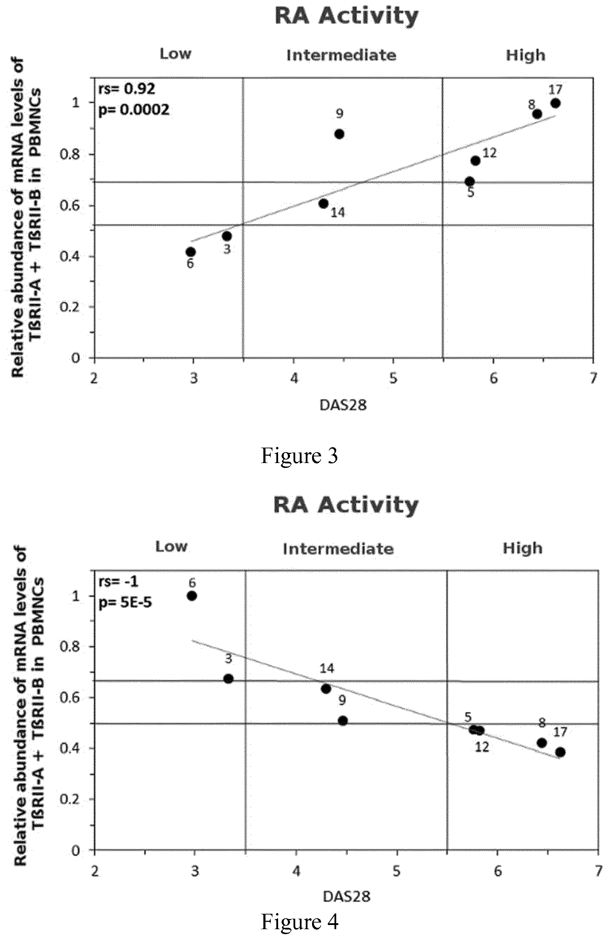

Figure 3 shows the graphs for the positive correlation between relative abundances of TβRII-A + TβRII-B mRNA's in PBMNCs, and the DAS28 score. The numbers correspond to the number assigned to each volunteer involved in the study. The analysis of the correlations was carried out by employing Spearman's test, with the use of the OriginPro 8.5.1 software (Origin Lab Corporation, Northampton, MA, USA). Values of $p < 0.05$ were considered to have statistical significance. rs: Spearman's correlation coefficient.

Figure 4 shows the positive correlation between the quotient between the relative abundances of TβRII-Se's mRNA and the addition of TβRII-A and TβRII-B in PBMNCs, and the DAS28 score. The numbers correspond to the number assigned to each volunteer involved in the study. The analysis of the correlations was carried out by employing Spearman's test, with the use of the OriginPro 8.5.1 software (Origin Lab Corporation, Northampton, MA, USA). Values of $p<0.05$ were considered to have statistical significance. rs: Spearman's correlation coefficient.

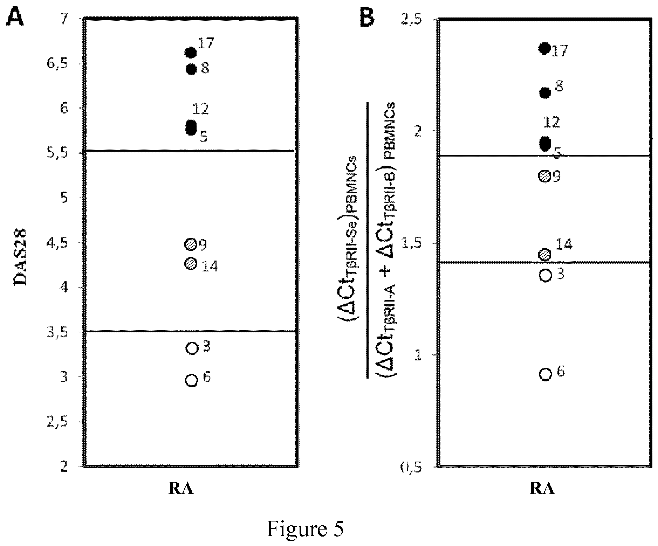

Figure 5 shows (A) Activity of the disease in patients with RA according to DAS28. (B) Activity of the disease according to the quotient between TβRII-Se's mRNA levels and the addition of TβRII-A and TβRII-B levels in PBMNCs (method of present invention) Black dots: high activity according to DAS28. Grey dots: moderate activity according to DAS28. Empty dots: low activity according to DAS28

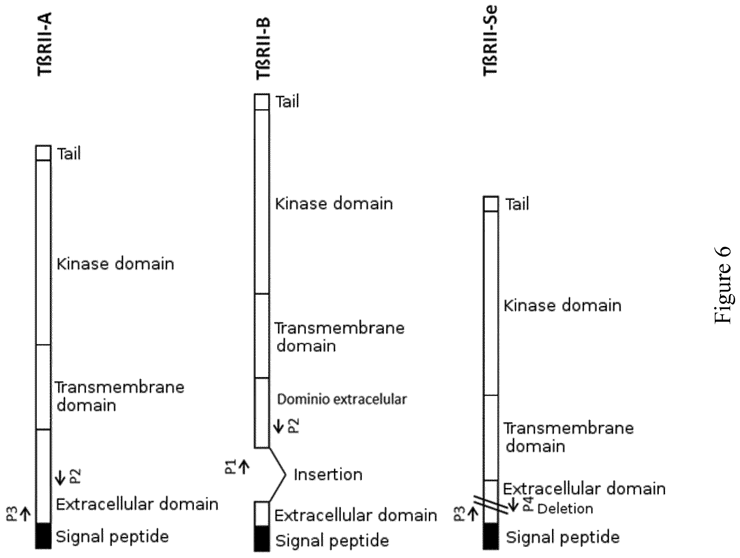

Figure 6 is a schematic depiction of the sites where the primers used in RT-qPCr are adhered. Amplified product TβPII-A (SEQ ID N° 1) with primers P3 (SEQ ID N° 2) and P2 (SEQ ID N° 3): 218pb. Amplified product TβRII-B (SEQ ID N° 4) with primers P1 (SEQ ID N° 5) and P2 (SEQ ID N° 3): 233pb. Amplified product TβRII-Se (SEQ ID N° 6) with primers P3 (SEQ ID N° 2) and P4 (SEQ ID N° 7): 137pb.

Detailed description of present invention

For the purposes of the present invention, "RA" are the initials of "rheumatoid arthritis".

By "addition of relative abundance of monocytes and lymphocites" it is meant the TβRII-A, TβRIIB orTβRII-Se's mARN level in monocytes and lymphocytes substracted from the value of the reference known gene.

For the purposes of the present invention the initials PBMNCs are defined as the mononuclear cells (monocytes and lymphocytes) isolated from peripheral blood.

With the term "TβRII-A, TβRIIB or TβRII-Se isoforms", splicing variants of TGF-β's Type II receptor that code for the corresponding polypeptides are defined.

This method presents several advantages such as, for example. that it is not necessary to segregate monocytes (plastic adherent cells) from other mononuclear cells (non- adherent to plastic): all of the mononuclear cells can be used, what entails the subsequent reduction of the processing time in 18 hours and without the need for complex equipment (laminar flow bench, $CO_2$ incubator) or the handling by experienced personnel.

When TβRII-A+TβRII-B are analyzed in all of the mononuclear cells, false positives have occurred that through the inclusión of TβRII-Se, place themselves again within the corresponding activity group according to DAS28. Besides, with the present method better correlations and more significant from a statistical viewpoint have been achieved, than with the previous method.

The present method allows for the identification of different activity levels of rheumatoid arthritis, by means of the quantification in peripheral blood mononuclear cells, of the mRNA of three isoforms of the Type II receptor of TGF-β. The isoforms are TβRII-A, TβRII-B and TβRII-Se, or the detection of said isoforms as polypeptides, where the TβRII-A sequence is SEQ ID N° 8 or sequences having between 90% and 99% of identity; the TβRII-B sequence is SEQ ID N° 9 or sequences having between 90% and 99% of identity and the TβRII-Se sequence is SEQ ID N° 10 or sequences having between 90% and 99% of identity. In a preferred embodiment, the quantification method is carried out by means of the RT-qPCR technique.

In a preferred embodiment, the present method comprises the obtainment of peripheral blood and a later purification or isolation of the mononuclear cells by means of, for example, density gradient centrifugation (for example, Ficoll-Paque) or by any other known method. For example, known methods are a separation by means of flow cytometry (cell sorting) and magnetic immunoseparation. Afterwards, the isolated cells can be incubated at 37 °C with 5% of $CO_2$ for 12- 16 hours. The adhered cells are the monocytes, while the non-adhered cells are the lymphocytes. Monocyte and lymphocyte (BMNCs) mARN is purified by means of known methods. Finally. The cDNA is generated from the mRNA total of monocytes and lymphocytes and it is quantified by means of PCR in real time (RT-qPCR) with specific primers. Lastly, the corresponding calculations are carried out.

After the adherent (monocytes) and non -adherent (lymphocytes) PBMNCs purification was performed, purity was assessed by means of flow cytometry, use being made of cell granularity (expressed as side scattering of light or SCC) and cell size (expressed as forward scattering of light or FSC). Said measurement varied between 52 % and 87 % for lymphocytes from volunteers with RA, and between 36 % and 89 % for monocytes from volunteers with RA (Table 1).

Table 1

| ID and sex of patients | Lymphocyte purity (%) | Monocyte purity (%) |
| --- | --- | --- |
| 03, F | 51.77 | 58.63 |
| 06, M | 78.96 | 43.31 |
| 09, M | 73.19 | 82.24 |
| 12, F | 74.37 | 67.48 |
| 14, F | 83.80 | 89.35 |
| 08, F | 73.59 | 35.84 |
| 17, F | 86.88 | 81.77 |
| 05, F | 71.49 | 69.15 |

Table 1. Purity of lymphocytes and monocytes isolated from peripheral blood of volunteers having RA established by flow cytometry. The numbers by the volunteers correspond to the ones assigned to said volunteers for the trial. F; female; M: male.

In Figure 1, representative flow cytometry diagrams and the purity percentage of lymphocyte and monocyte populations are depicted.

With the purpose of assessing the possibility of the develpment of a method that contribute with objective parameters to determine the activity of AR or other autoimmune diseases, the relative levels of mARN from the diverse isoforms of TβRII where correlated, where the mARN was isolated from PBMNCs (lymphocytes and monocytes) pertaining to RA patients that showed clinical and biochemical parameters of the disease.

Under the light of what is disclosed here. It is understood that it is also possible to employ methods that measure isoform levels as polypeptides that are soluble or are joined to membranes. All of those methods are included within the scope of the present invention,

In RA patients it could be seen that their levels of mARN of TβRII-A and TβRII-B in PBMNCs are positively, and in a significant way, correlated with the quantity of de tender joints and the quantity of swollen joints (Figure 2).

The three-parameter DAS28 equation is an index routinely used to determine RA activity and includes the quanityt of tender joints (TJC), the quantity of swollen joints (SJC) -both determined in a total quantity of 28 defined joints-and as (SJC) -ambas determinadas sobre un total de 28 articulaciones definidas-, and the erythrocyte sedimentation rate (ESR).

$$DAS28 = 0.56 \sqrt{NAD} + 0.28 \sqrt{NAI} + 0.70 \, (\ln ESR)$$

It can be expected that if the correlations between the mRNA levels of TβRII-A and TβRII-B in both populations and the number of tender and swollen joints are significant, the same happens with those between the same mRNA and DAS28, since both of these clinical determinations are contained in the definition of DAS28. It is important to remark that the ESR is also contained in the DAS28 score, but this is a non especific variable that may be affected by other factors, not only from swelling. Because of this, the ESR is generally used together with other determinations of the disease. As expected, DAS28 values correlate positively with mRNA values of both isoforms of the receptor in PBMNCs (TβRII-A and TβRII-B) (Figure 3).

By using the DAS28 score, three degrees of disease activity can be determined: low (values lower than or equal to 3.5), moderate (values higher than 3.5 and lower than, or equal to, 5.5) and high (values higher than 5.5) (Table 2).

Table 2

| RA Activity | Value DAS28 | Patient N° (value DAS28) |
|---|---|---|
| Low | ≤ 3.5 | 06 (2.97) <br> 03 (3.33) |
| Moderate | > 3.5 and ≤ 5.5 | 09 (4.46) <br> 14 (4.30) |
| High | > 5.5 | 05 (5.76) <br> 12 (5.82) <br> 08 (6.44) <br> 17 (6.62) |

Table 2. Classification criterion for RA activity according to DAS28. Here are included the patients involved in the trial in the corresponding category of RA according to their value of DAS28.

When the significant positive correlations obtained were analyzed in detail, it was seen that the mRNA level of TβRII-A and TβRII-B in PBMNCs from patients with RA is distributed in a greatly similar way in the three groups the disease is classified in according to DAS28. The mRNA levels of TβRII-A and TβRII-B in the PBMNCs from RA patients increase accordingly to the activity increase of the disease, there being a false positive occurrence corresponding to patient 9. However, when the quotient between the relative levels of TβRII-Se and the addition of the two remaining isoforms TβRII-A and TβRII-B (Figure 4) are included in the calculation, it is obrained a higher negative correlation ((rs=-1) and also more significant from the statistical viewpoint (p= 5,5 x 10$^{-5}$) than the one obtained using only the relative abundance of mRNA of TβRIIA plus TβRII-B, where patient 9 is positioned in the group of intermediate activity, in coincidence with the value of DAS28.

Therefore, from the aforesaid analysis the mRNA levels of the three isoforms of TβRII in the PBMNcs from RA patients as candidates to be assessed as new biomarkers for the disease, where selected. It could be determined that the quotient between the mRNA levels of TβRII-Se and the addition of the TβRII-A and TβRII-B levels in PBMNCs from patients with RA diminishes as the disease activity increases and they become distributed in three groups that coincide with the disease categories determined by the DAS28 values.

Then, cut-off values for the levels can be established in the result of the quotient between the mRNA values of TβRII-Se and the addition of mRNA levels of TβRII-A and TβRII-B, to employ this parameter as a biomarker for RA diagnosis, activity determination and prognosis.

The detection method for RA can be seen in Figure 5. In a preferred embodiment, the method is based on the use of the Ct parameter (*cycle threshold*) obtained by RT-qPCR, where ΔCt is the difference of Ct between the value gotten with each one of the isoforms and the Ct of the reference gene, in this case GAPDH. Therefore, the calculation used to perform this approximation is the quotient between $\Delta Ct_{T\beta RII-Se}/(\Delta Ct_{T\beta RII-A}+\Delta Ct_{T\beta RII-B})$, being $\Delta Ct_{T\beta RII-B-Se}$ = $Ct_{T\beta RII-Se}$ - $Ct_{GAPDH}$; $\Delta Ct_{T\beta RII-A}$ = $Ct_{T\beta RII-A}$ - $Ct_{GAPDH}$; $\Delta Ct_{T\beta RII-B}$ = $Ct_{T\beta RII-B}$ - $CtGAPDH$, respectively

In the example, GAPDH has been used as a reference gene, but the experts in this art know that in order to carry out the method of the present invention, any known reference gene may be used.

Thus it can be established that patients having an isoform quotient higher than 1.8 could be considered as having a high activity of the disease; between 1.8 and 1.4, intermediate activity and below 1.4, low activity.

These indexes are mandatory for the general practitioner, taking into account that they show the degree of activity of the disease, thus permitting the making of therapeutic decisions as a function of objective evidence. It is also possible to monitor the therapeutic response and establish an evolution prognosis. The method disclosed by the present invention clearly is an objective method.

The method according to the present invention allows for the determination of rheumatoid arthritis from, for example, the detection of the levels of messenger RNA, but it is also possible from the measurement of the three codified isoforms of the TGF-β type II receptor. Because of the high correlation between the transcription of genes of interest and the degree of progress of the pathology, the present invention proposes an efficient and reliable method for the diagnosis and follow-up of the disease.

The present method permits:

- The diagnosis and determination of RA activity and other autoimmune diseases, such as systemic lupus erythematosus, systemic sclerosis, polydermatomyositis, vasculitis and seronegative spondyloarthropathies.

- To establish the degree of activity of the disease by means of an objective method.

- To establish prognoses.

- A marker of treatment effectiveness.

- The following up of the disease.

- The use of quantification methods such as, for example RT- PCR

- The diagnosis is made based on peripheral blood samples.

This invention is best illustrated by the following examples, which should not be construed as a limitation imposed on the scope of the invention: on the contrary, it must be clearly understood that other embodiments, modifications and equivalents of the present invention can be resorted to, because the reading of the present specifications may suggest that to the experts in this art without departing from the spirit or the scope, or both at the same time, of the enclosed claims.

**Examples**

**Example 1: Samples from patients with rheumatoid arthritis (RA)**

The samples were taken at the Instituto Medico CER, Quilmes, Buenos Aires Province, Argentina, and the clinical and biochemical studies were performed at that same Institution. All of the volunteers with RA (N=8) included in this study signed an informed consent prior to the taking of data and samples. The trial was approved by the bioethics institutional commission of the Servicio de Reumatología del Instituto Medico CER, Quilmes, Buenos Aires (Rheumatology Department of the aforementioned Instituto CER) and the Health ministry of Buenos Aires Province. The criteria for the inclusion of volunteers in this trial were that those volunteers would be willing to give their previous informed consent, were older than 18 years of age and met the ARA 1987 criteria (Arnett, F. C. et al., Arthritis Rheum. 1988 31 (3):315-24. Criteria for exclusion were diseases and their concomitant medication that might generate a bias of the interpretation of results, such as the use of biological drugs.

Information on age, sex, evolution time of the disease, concomitant diseases, medication for RA, concomitant medication was gotten, and the erythrocyte sedimentation rate (ESR) was measured. On diseased volunteers it was determined the number of tender joints, radiological injuries and bone mineral density in lumbar spine and femoral neck. Data from three parameters, DAS28 (*Disease Activity Scores using 28 joint counts*), HAQ (*Health Assessment Questionnaire*) and VAS (*Visual Analogue Scale*) were also obtained.

The data and clinical and biochemical determinations of the volunteers are summarized in the Table 3, below:.

Table 3

| Characteristics | RA (n=8) |
|---|---|
| *Demographic* | |
| Age, years | 50.2 (23-78) |
| Sex F/M | 6/2 |
| Duration of disease, years | 11.58 (0.25-21) |
| *Clinical determinations* | |
| Number of tender joints | 9.2 (0-20) |
| Number of swollen joints | 7.6 (0-19) |
| DAS28 | 4.77 (2.58-6.65) |
| HAQ | 1.102 (0.125-2800) |
| VAS pain in patient, mm | 43.6 (20-72) |
| VAS activity of patient, mm | 43.6 (29-78) |
| VAS physician, mm | 47 (5-80) |
| *Laboratory analytical parameter* | |
| ESD, mm | 36.3 (9-65) |
| *Treatment with drugs* | |
| NSAID | 5/8 |
| DMARD | 6/8 |

Table 3. The values correspond to means and, inside parentheses, minimums and maximums. F: female; M: male; ESD: erytho cell sedimentation; NSAID: , *Non-Steroidal Anti-Inflammatory Drugs*; DMARD: *Disease-Modifying Anti-Rheumatic Drugs;*

**Example 2: Isolation of leukocytes and separation of the total of lymphocytes, and of monocytes.**

From heparinized peripheral blood, a centrifugation per gradient was carried out with Ficoll-Paque™ PLUS (GE Healthcare Bio-Sciences AB), after which a fraction containing peripheral blood mononuclear cells (PBMCs) was obtained. The number of PBMCs was quantified in A Neubauer chamber by means of an exclusion method with trypan blue.

The total quantity of lymphocytes and the monocytes of patients with RA were separated from the PBMCs on the basis of their differential properties of adherence to plastic. In order to do that, the PBMCs were cultured for 2 hours in RPMI medium supplemented with 10% of human serum (HS), after which the cell supernatant containing lymphocytes mainly, was collected. The monocytes, adhered to the culture vial, were grown for 16 hours, after which time they were collected by means of treatment with trypsin- EDTA. The purity of both populations was determined by flow cytometry with a BD FACSCalibur™ (BD, USA equipment), use being made of cell size (FSC) and cell complexity (SSC). The viable cells from both populations were quantified by the method of exclusion wirh trypan blue in a Neubauer chamber. They were kept at -80 °C in lisis *buffer* for the extraction of RNA, until they were used.

**Example 3: Flow cytometry assay**

For the analysis by flow cytometry, a minimum of 5 x$10^4$ quantified viable cells were employed in a Neubauer chamber by the exclusión method with trypan blue. The cells were re-suspended in 100 $\mu$l of fixing solution. The measurements were performed with a FACSCalibur (BD Biosciences, USA) equipment and the analysis of the data obtained was done with the WinMDI program.

**Example 4: Quantitative RT-PCR (RT-qPCR)**

Extraction of RNA. The RNA was isolated from $10^4$-$10^6$ cells, to do which the comercial kit Absolutely RNA® Miniprep Kit (Stratagene, La Jolla, CA, USA) or the comercial kit SV Total RNA Isolation System (Promega, WI, USA), were used, the instructions from the manufacturers having been followed in each case. The purified RNA was re-suspended in 50 $\mu$l of elution *buffer* or nuclease- free water, according to the recommendations of the manufacturer of each kit, and it was quantified, a spectrophotometer BioPhotometer (Eppendorf, Germany) having been used, and it was kept at -80 °C until the moment to use it.

Generation of complementary DNA (cDNA). Reverse transcription reactions were carried out in a final volume of 40 μl composed of 1.5 μg of RNA, 0.2 μM of *primers* OligodT15 (Promega, WI, USA) and 200 U of reverse transcriptase from M-MLV (Promega, WI, USA), the manufacturer's instructions having been followed. The cDNA generated was kept at -80 °C until its use.

RT-qPCR reactions were carried out using a Mx3005P™ (Stratagene, USA) equipment. The reactions were performed in a final volume of 10 μl, composed of 2 μl of cDNA, Fast Start Universal SYBR Green Master (ROX) 1X (Roche Applied Science, Mannheim, Germany), 300 nM of direct and reverse primers for the amplification of TβRII-A, TβRII-B and GAPDH[P3 (SEQ ID N° 2) and P2 (SEQ ID N° 3), P1 (SEQ ID N° 5) and P2 (SEQ ID N° 3) and GAPDH-F (SEQ ID N° 11) and GAPDH-R (SEQ ID N° 12)] and 200 nM for the amplification of TβRII-Se [P3 (SEQ ID N° 2) and P4 (SEQ ID N° 7)]. In each assay the samples were analyzed in triplicate..

As a control normalizing gene, primers that amplify the constitutive gene of Glyceraldehyde 3-phosphate dehydrogenase (GAPDH), the sequences of which are: GAPDH-F: 5' GTCAGTGGTGGACCTGACC 3' (SEQ ID N° 11), GAPDH-R: 5' TGAGCTTGACAAAGTGGTCG 3' (SEQ ID N° 12), were utilized.

The conditions for amplification were: 1 cycle of 15 minutes at 95°C and 40 cycles of 10 seconds at 95 °C and 30 seconds at 62 °C. Afterwards, a dissociation curve to assess the presence of non specific amplification products was carried out. The sequences of the primers employed were: P1: 5'CTGTAATAGGACTGCCCATC 3'(SEQID N° 5), P2: 5' TCTCTAGTGTTATGTTCTCGTC 3' (SEQ ID N° 3), P3: 5' GCACGTTCAGAAGTCGGTTAA 3' (SEQ ID N° 2), P4: 5' GCACTTTGGAGAAGCAGGATT 3' (SEQ ID N° 7). For the specific amplification of the different isoforms of TβRII, diverse combinations of these primers were used. For TβRII-A, the primers P2 and P3 were used; for TβRII-B, P1 and P2 and for TβRII-Se, the primers P3 and P4 (see Figure 6).

Statistics: The statistical analysis of the data coming from the RT-qPCR's was performed by means of the InfoStat version 2010 software (Di Rienzo, J. A. *et al.,*http://www.infostat.com.ar).

The analysis of the correlations was performed through Spearman's test. To carry out these tests the OriginPro 8.5.1 (Origin Lab Corporation, Northampton, MA, USA software was utilized. In all of the cases, values of $p < 0.05$ were considered statistically significant.

## Claims

1. A method to determine the activity level of autoimmune diseases, **characterized in that** it comprises

   a) providing a biological sample;
   b) determining the level of the TβRII-A, TβRII-B y TβRII-Se isoforms **in that** biological sample
   c) calculating the activity level by performing the quotient between the TβRII-Se level and the level of the addition of TβRII-A to TβRII-B.

2. The method according to claim 1, **characterized in that** the said biological sample comprises isolated mononuclear circulating blood cells.

3. The method according to claim 1, **characterized in that** the said biological sample comprises the circulating TβRII-A, TβRII-B and TβRII-Se isoforms.

4. The method according to claims 1 and 2, **characterized in that** the level of the TβRII-A, TβRII-B and TβRII-Se isoforms from the biological sample comprises the mRNA level of said isoforms.

5. The method according to claims 1 and 2, **characterized in that** the level of the TβRII-A, TβRII-B and TβRII-Se isoforms from the biological sample comprises the polypeptide level of the sequences SEQ ID N° 8, SEQ ID N° 9 and SEQ ID N° 10 or sequences having at least a 90% of identity with said sequences.

6. The method according to claim 1, **characterized in that** the stage b) is carried out by means of RT-qPCR.

7. The method according to claim 1, **characterized in that** the autoimmune disease is selected from the group consisting of rheumatoid arthritis, systemic lupus erythematosus, systemic sclerosis, polydermatomyositis, vasculitis and seronegative spondyloarthropathies.

8. A kit to determine the activity level of autoimmune diseases, **characterized in that** it comprises:

   i) specific primers to amplify the TβRII-A, TβRII-B y TβRII-Se isoforms,
   ii) reverse transcriptase and Taq polymerase enzymes, and
   iii) media to reveal said isoforms.

9. The kit according to claim 8, **characterized in that** it comprises media for the obtainment and isolation of blood mononuclear cells.

10. The kit according to claim 8, **characterized in that** the autoimmune disease is selected from the group consisting of rheumatoid arthritis, systemic lupus erythematosus, systemic sclerosis, polydermatomyositis, vasculitis and seronegative spondyloarthropathies.

11. The kit according to claim 8, **characterized in that** the revelation means are selected from the group consisting of fluorescence, colorimetry and luminescence.

Figure 1

Figure 2

Figure 3

Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AR P20130104170

- AR 093466A19

**Non-patent literature cited in the description**

- **ARNETT, F. C. et al.** *Arthritis Rheum.,* 1988, vol. 31 (3), 315-24